# EUROPEAN PATENT SPECIFICATION

(11) **EP 2 605 718 B1**
(45) Date of publication and mention of the grant of the patent: **04.03.2015**
(21) Application number: 11817855.7
(22) Date of filing: 04.08.2011
(51) Int. Cl.: A61B 18/18, A61N 1/32, A61B 18/14

(54) **ELECTROMAGNETIC ENERGY APPLICATOR FOR PERSONAL AESTHETIC SKIN TREATMENT**
ELEKTROMAGNETISCHER ENERGIEAPPLIKATOR FÜR PERSÖNLICHEN ÄSTHETISCHEN BEHANDLUNG DER HAUT
APPLICATEUR D'ÉNERGIE ÉLECTROMAGNÉTIQUE POUR UN TRAITEMENT ESTHÉTIQUE PERSONNEL DE LA PEAU

(30) Priority: 19.08.2010 US 375054 P
(43) Date of publication of application: 26.06.2013
(73) Proprietor: Syneron Medical Ltd., 20692 Yoqneam Illit (IL)
(72) Inventor: ECKHOUSE, Shimon, 34980 Haifa (IL); FLYASH, Lion, 17512 nazareth Illit (IL); VAYNBERG, Boris, 30900 Zichron Yaakov (IL)
(74) Representative: O'Neill, Brian
(86) International application number: PCT/IL2011/000630
(87) International publication number: WO 2012/023129

(56) References cited:
- US-A1- 2004 002 705
- US-A1- 2007 179 482
- US-A1- 2007 191 827
- US-A1- 2010 016 761
- US-A1- 2010 179 531
- US-A1- 2010 179 531
- US-A1- 2010 191 238
- US-A1- 2010 204 695

## Description

### FIELD OF TECHNOLOGY

The present apparatus is related to the field of personal aesthetic procedures and in particular to cosmetic skin treatment procedures.

### BACKGROUND

Skin tightening or wrinkle reduction, removal of skin lesions and reduction of subcutaneous fat or adipose tissue, are aesthetic treatments for which there is a growing demand. Types of available aesthetic therapy commonly include the application of different light sources , radio frequency energy and sometimes ultrasound energy

The electromagnetic energy is typically delivered to a target segment of the skin of a recipient by selecting a contact element that is compatible with the treated segment size. Alternatively, a plurality of contact elements may be utilized, in which the plurality of elements contact discrete points of the target segment of the skin. In the latter case, the healing period is typically shorter. Although both modes of treatment are effective, the use of multiple contact elements treating discrete points or fractions of a target segment effectively tightens the skin, reduces wrinkles, and improves the skin appearance. In recent years, noninvasive, non-ablative aesthetic skin treatments have been introduced and may replace ablative skin treatment procedures in the future. In non-ablative skin treatment thermal energy induces certain tissue modification and in particular collagen modification in the dermis. Currently non-ablative skin treatment is used for skin tightening, scar removal, acne treatment, and other aesthetic procedures typically performed in an ambulatory environment.

In non-ablative skin treatment light and/or radiofrequency (RF) energy is deposited 100-2500 µm below the skin surface, where the energy does not affect the epidermis and the skin layer in which most of the skin aging processes occur. With no epidermal wound, there is almost no recovery period and thus no interruption of daily life routines. Transient erythema or mild edema, are the only known side effects and those disappear a few hours after the treatment. The efficiency of the non-ablative treatments is lower than the one of ablative treatments; however, non-ablative skin treatments also stimulate new collagen production and repair tissue defects.

Since there are no side effects and the procedure does not leave wounds requiring a long healing period, the non-ablative treatment is associated with little or no downtime and unlike the ablative skin treatment, which requires professional supervision, non-ablative skin treatment may be used by a lay user in a home environment at a time most convenient for him/her to perform a treatment session such as, for example, skin tightening and wrinkle reduction associated with collagen remodeling.

Both light and Radio Frequency (RF) energy types may be used for these procedures. RF however, does not scatter and, penetrates deeper into the dermis and causes negligible heat sensation on the skin surface.

RF energy is conducted to skin through electrodes. With proper design of RF applying electrodes, RF energy power setting and application time the energy may be accurately conducted to the desired target tissue. For example, the energy application time and power may be shorter than skin thermal relaxation time further simplifying the non-ablative skin treatment. The employment of an applicator that includes disposable parts for electromagnetic radiation skin treatment also simplifies and facilitates aesthetic treatments in a home environment at a time most convenient for the user to perform a treatment session. Document US-A-2010/016761 discloses the most relevant prior art.

### BRIEF SUMMARY

According to the present invention, there is provided an apparatus as specified in claim 1. An apparatus for personal aesthetic skin treatment by RF voltage. The apparatus includes an assembly of individual electrodes operative to contact fractions of the skin and deliver to each contact RF voltage. The voltage may be a test voltage enabling determination of the quality of the contact between each of the electrodes and the skin and skin treatment voltage. The treatment voltage heats the skin and is applied only to electrodes being in proper contact with the skin. Appropriate skin treatment protocols are stored in the apparatus and the selected skin treatment protocol sets the number of electrodes to which RF voltage and the magnitude of the voltage applied. The selected protocol and skin treatment parameters ensure safe non-ablative skin treatment.

Typically, the electrodes are assembled on a common substrate or carrier that may be a reusable or disposable carrier. In course of the treatment the applicator with the carrier is applied to the skin in a patch-like step motion or moved in a sweep like movement over the treated skin segment.

### GLOSSARY

The term "carrier" in the context of the present disclosure means a substrate having an array of voltage to skin application elements or electrodes. The electrodes may be in the form of one or more rows of voltage-to-skin application elements, a two dimensional array or matrix of voltage-to-skin application elements and a three dimensional shape substrate having on its external surface voltage-to-skin application elements.

The terms "electrodes", "conductive elements", "contact elements" and "voltage to skin application elements" are used interchangeably in the present disclosure and mean elements operative to receive voltage from a source such as, for example, an RF voltage generator and apply the received voltage to the skin, or serve as a return electrode.

The term "skin treatment" as used in the present disclosure includes aesthetic or cosmetic treatment of various skin layers such as stratum corneum, dermis, epidermis, skin rejuvenation procedures, pigmented lesions removal, and such procedures as collagen shrinking or destruction.

The terms "RF voltage" and "RF power" are used interchangeably in the present disclosure. The mathematical relation between these two parameters is well known and knowledge of the value of one of them enables easy determination of the value of the other parameter.

The term "a large segment of skin" as used in the context of the present disclosure, means a segment of skin dimensions of which exceed the dimensions of the surface of the carrier, or circumference of the surface of the contact electrode or electrodes carrier.

### BRIEF DESCRIPTION OF THE DRAWINGS

Various embodiments of the present apparatus, including method and apparatus embodiments, are disclosed and presented, by way of nonlimiting examples only, with reference to the accompanying drawings, wherein like numerals depict the same elements throughout the text of the specifications. The present apparatus and skin treatment method will be understood and appreciated more fully from the following detailed description, taken in conjunction with the drawings in which:
FIG. 1 is a simplified illustration of the present apparatus for personal aesthetic skin treatment;
FIGS. 2A, 2B and 2C are plan view and a cross section view simplified illustrations of an exemplary aesthetic skin treatment apparatus with an asymmetric carrier such as that shown in FIG. 1;
FIG. 3 is a planar view simplified illustration of another exemplary embodiment of a carrier with skin contacting elements and LEDs in accordance with the present method and apparatus;
FIG. 4 is a planar view simplified illustration of an additional exemplary embodiment of a carrier with skin contacting elements and openings for LEDs in accordance with the present method and apparatus;
FIG. 5 is a planar view simplified illustration of another exemplary embodiment of the tip with LEDs in accordance with the present method and apparatus;
FIG. 6 is a planar view of an additional exemplary carrier with skin contacting elements in accordance with the current method and apparatus;
FIG. 7 is a planar view simplified illustration of another exemplary embodiment of a carrier with skin contacting elements in accordance with the current method and apparatus;
FIGs 8A and 8B are schematic illustrations of full and insufficient contact of the electrode with a segment of skin;
FIG 9 is a schematic illustration of an exemplary skin treatment process with the present apparatus;
FIG. 10 is a simplified illustration of the present apparatus for personal aesthetic skin treatment with a tip implemented in shape of a body with rotational symmetry; and
FIG. 11 is a simplified illustration of the display of the present apparatus indicating electrode status in course of the aesthetic skin treatment process.

### DETAILED DESCRIPTION OF EXEMPLARY EMBODIMENTS

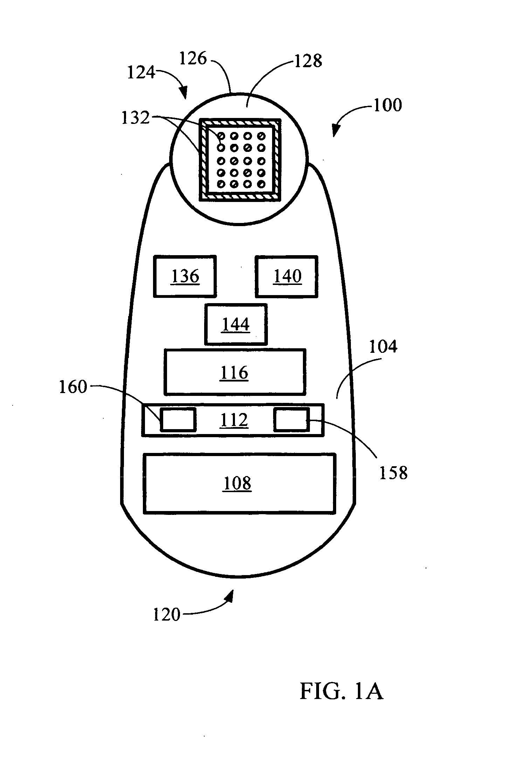

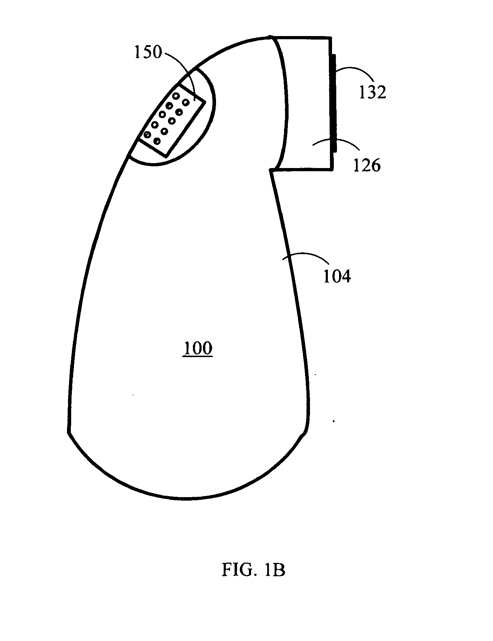

Reference is made to FIGS. 1A and 1B, which are a front and side view simplified illustration of the present apparatus for personal aesthetic skin treatment. As shown in FIG. 1A, apparatus 100 includes a palm held case 104 containing a source of power 108, a controller 112 and an RF voltage generator 116. The source of power 108 may be one or more of regular batteries that are disposed upon depletion or one or more of rechargeable batteries. The proximal end 120 of apparatus 100 may have an inlet accepting an electric network cable for battery charge. Distal end 124 of apparatus 100 includes a tip 126, which may be a removable tip. Each tip 126 either includes a carrier 128. Each carrier includes a plurality or an assembly of skin contacting elements or electrodes 132 to be described in detail below. Carrier 128 is such that it supports easy mounting and exchange of different disposable or reusable carriers 128. A number of different sensors or sensing mechanisms such as an electrode-to-skin quality of contact detecting mechanism 136, skin and or electrode temperature sensors 140 may be located close to carrier 128 or on carrier 128 and may be associated with one or more of electrodes 132.

Apparatus 100 is intended for personal aesthetic skin treatment and in operation it heats skin segments and/or volumes in contact with the electrodes and between the operating electrodes. As will be explained below, operational parameters of apparatus 100 are set to heat the skin only and not to cause skin ablation. Nevertheless, skin ablation may be accidentally initiated by improper or insufficient electrode to skin contact or occasional skin defects.

Controller 112 could include a mechanism 158 having a memory 160 that stores and retrieves different skin treatment protocols and sets the skin treatment parameters according to the retrieved skin treatment protocol, which could be a non-ablative skin treatment protocol. Memory device 160 may communicate with controller 112. Controller 112 communicates with each of the sensors, receives from the sensors treatment status signals. Memory device 160 stores treatment protocols or instructions which, when executed by the controller, cause the controller to detect the quality of the contact between the electrode and the skin; determine the number of electrodes to be operated simultaneously; retrieve appropriate skin treatment protocol and set the treatment parameters according to the retrieved a skin treatment protocol. The selected protocol and skin treatment parameters ensure safe skin treatment, which may be non-ablative skin treatment. Additionally, controller 112 in course of treatment may adjust the skin treatment parameters according to the received signals if deviations of the set or predetermined protocol exist. For safety reasons apparatus 100 also includes skin treatment safety measures such as a skin ablation detecting mechanism 144 operative to monitor the ongoing skin treatment status and detect situations in which ablation has been initiated. When such a situation is detected the information is communicated to controller 112 that, in turn, adjusts the skin treatment parameters accordingly to maintain a non-ablative skin treatment. RF generator 116 has a plurality of exists and provides RF voltage independently and individually to each of the skin contacting elements or electrodes 132. The voltage may be a test voltage enabling determination of the quality of the contact between each of the electrodes and the skin and skin treatment voltage and/or a treatment voltage.

Selection of the number of simultaneously operating individual electrodes enables addressing and RF voltage delivery to the entire assembly or array of skin contacting elements or electrods 132, a group of the array of skin contacting elements 132 and as noted to each of the skin contacting elements 132 individually. Depending on the configuration of carrier 128 RF electrodes 132 may be configured to operate in a monopolar or bipolar mode. Apparatus 100 may also include a display 150 operative to provide to the user information on the skin treatment process progress and/or a graphic map of the last treatment application. Display 150 may be an Organic Light Emitting Diodes (OLED) display, a Liquid Crystal Display (LCD) or a matrix of Light Emitting Diodes (LED) that among others could display for each electrode electrode-to-skin contact status.

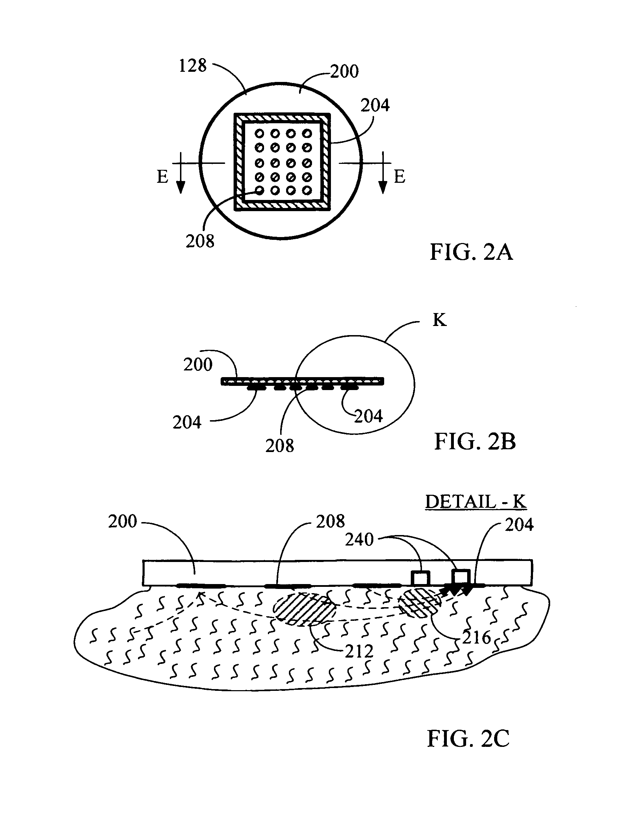

FIGS. 2A, 2B and 2C are plan view and cross section view simplified illustrations of an exemplary aesthetic skin treatment apparatus with an asymmetric carrier such as that shown in FIG. 1. Asymmetric carrier 128 (FIG. 2A) includes a substrate 200, an assembly or array of miniature voltage-to-skin applying elements or electrodes 208 commonly surrounded or flanked by a return electrode 204, which could completely or partially surround electrodes 208 . Although shown as a rectangle, the return electrode may be a circle, a segment of a circle, or simply one or more electrode strips. The term "asymmetric" carrier is based on the asymmetric distribution of impedances along a path of current between RF electrodes 208 and the return electrode 204 as shown in FIG. 2C. A high impedance exists below RF electrode (electrode-to-skin impedance) 208, for example, in a volume of skin designated the reference numeral 212, disposed in series with a low impedance which exists in the return path to the return electrodes (return electrode-to-skin impedance), for example, in a volume of skin designated the reference numeral 216.

In the asymmetric carrier configuration such as that shown in FIG. 2A, volume 216, disposed between the outermost electrodes 208 and return electrode 204, is where the return current is largest. This current heats skin tissue adjacent to return electrode 208, such as volume of skin 216. Skin and or electrode temperature sensors 240 optionally could be located on carrier 200 closer to the "hotter" skin segments and may be associated with one or more of electrodes 204.

In the currently used aesthetic skin treatment asymmetric applicator tip carrier 200 the return path between the outermost electrodes (that could have a pin type shape) 208 and return electrode 204 is the shortest current path having the lowest return impedance. Because of this a greater amount of RF power is delivered along this short return path, again, potentially generating a higher temperature in the adjacent skin segment or volume than in the rest of the treated skin segment or volume. Voltage-to-skin applying elements or electrodes may be produced by different methods. Typically, methods used in printed circuit board production may be suitable for voltage-to-skin applying elements or electrodes production. These methods enable low cost production of a large amount of carriers populated by electrodes. Depending on the type of processing and material deposition the voltage to skin applying elements may be flat, protruding from the surface on few microns, few tens of a millimeter, or more as desired. By proper selection of the metal deposition process the voltage to skin applying elements may be made of spherical or higher order shape. The substrate 200 of the carrier 128 on which the electrodes 204 and 208 reside is common to all electrodes and may be made of a variety of materials, typically insulating materials. Non-limiting example of a suitable material includes polyimide film, paper, or similar material, with a thickness of 0.5mil to 60mil (12.5 micron to 1500 micron). Carrier 128 is configured to allow quick attachment to apparatus 100.

Depending on the desired use the carrier may be implemented as a reusable or disposable carrier. When the same person is repeatable using the apparatus it may prefer a reusable carrier. If more than one person employs the apparatus, a disposable carrier may be preferred.

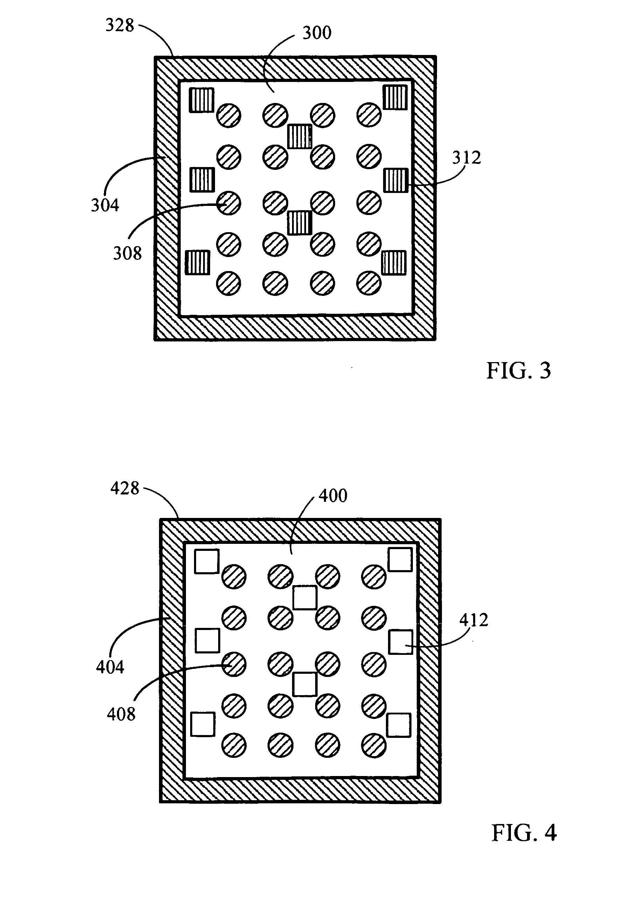

FIG. 3 is a planar view of another exemplary carrier with skin contacting elements and LEDs in accordance with the current method and apparatus. Carrier 328 includes a substrate 300, an array of miniature voltage-to-skin applying elements or electrodes 304 commonly or at least partially surrounded or flanked by a return electrode 308 and a plurality of Light Emitting Diodes (LEDs) 312 dispersed on carrier 328. The LEDs may be located on a grid, although the distance between neighboring LEDs in both dimensions of the grid may be larger than the distance between the electrodes. The distance between LEDs 312 is typically selected to provide sufficient illumination level to the skin. LEDs 312 may be surface mounted or other devices and operate at a plurality of wavelength emitting Blue, Yellow, Red or other colors. It is known that red light with wavelengths of 630nm to 780nm is effective in skin tightening procedures. Optionally, LEDs 312 may operate in the spectrum range between 630nm to 780nm. Other wavelengths initiating skin stimulation and rejuvenation processes may be applied to the skin. The treatment by light may be applied simultaneously with the treatment by RF and the skin may be illuminated in continuous or pulse mode.

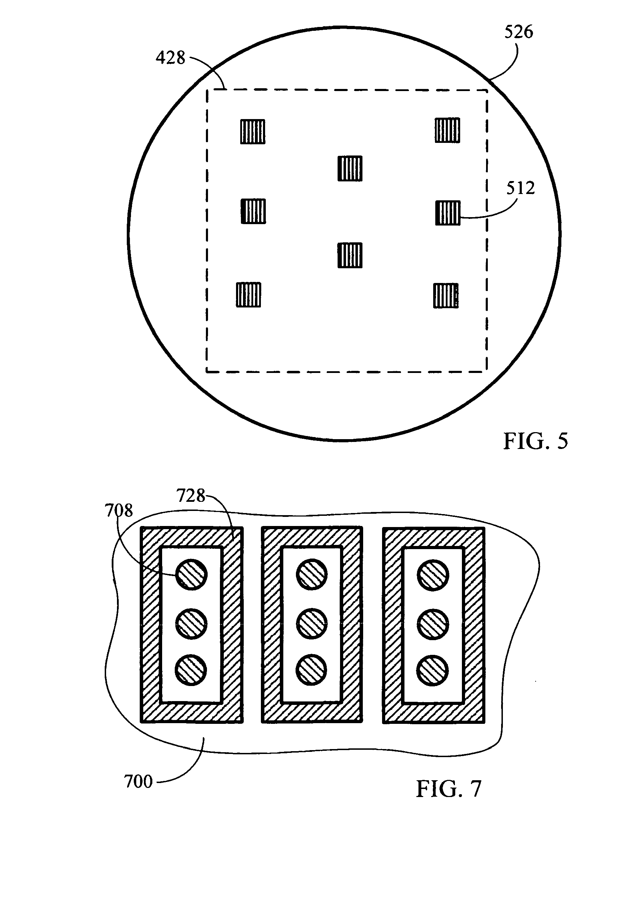

FIG. 4 is a planar view simplified illustration of an additional exemplary carrier with skin contacting elements and openings for LEDs in accordance with the current method and apparatus. Carrier 428 includes a substrate 400, an array of miniature voltage to skin applying elements or electrodes 404 commonly surrounded or flanked by a return electrode 408 and a plurality of openings 412. Openings 412 are configured and sized to accept LEDs 512 that as shown in FIG. 5 are located on tip 526 of the apparatus. Carrier 428 supports easy and accurate mounting on tip 526 of the skin treatment apparatus, since LEDs serve as registration pins.

FIG. 5 is a planar view simplified illustration of another exemplary embodiment of the tip with LEDs in accordance with the current method and apparatus. LEDs 512 are mounted on tip 526 that may serve as a reusable or fixed tip. For skin treatment carrier 428 (FIG. 4) is mounted on tip 526 (FIG. 5).

Temperature sensors 240 (FIG. 2) may be handled in a manner similar to the manner LEDs are used. The temperature sensors may be associated with the carrier and the electrodes or with the tip.

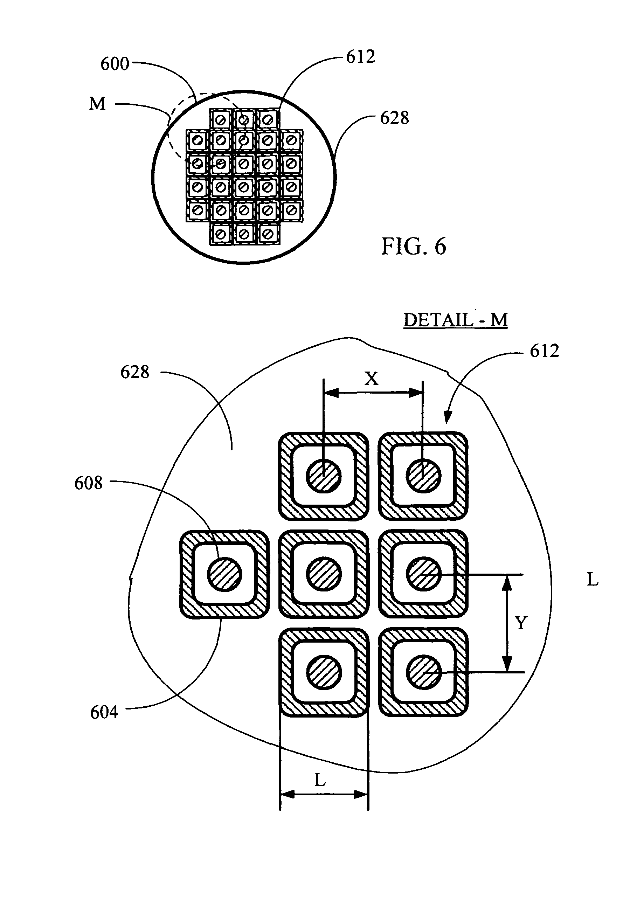

FIG. 6 is a planar view of an additional exemplary carrier with skin contacting elements in accordance with the present method and apparatus. In some skin treatments it may be desirable to affect a certain skin layer. Electrodes 612 located on substrate 628 of carrier 600 may include an active electrode 604 and a return electrode 608. It is known that the skin depth affected by the RF is roughly equal to half of the distance between the electrodes. (The particular carrier 600 bears electrodes 612 where the active electrode 604 is located in the center of a square return electrode 608 with side dimension L and the largest distance between the active electrode and the return electrode would be equal to half of the diagonal or 0.5x(√L). Other return electrode shapes may be used. For example the return electrode may be a circle with radius R, a segment of a circle or one or more conductive strips. Such electrode would affect a skin depth of about half of the radius R. By changing the shape and size of the return electrode one may change the skin depth affected by the RF. If all of the electrode shapes and size are the same, operation of each of the electrodes 612 affects or heats skin layers located on the same depth or equidistantly spaced from the skin surface or stratum corneum.

Generally, but not necessary, electrodes 612 may be located on a grid. The grid may have equal dimensions in both X and Y dimensions (square grid), although in some embodiments the distance in the X and Y directions may be different. The carrier electrodes 604 and 608 are configured such that in course of skin treatment one or more of the electrodes are in contact with the skin. Usually, a plurality of electrodes may be in contact with the skin at a plurality of skin locations. By changing the shape of the electrodes and distance between the electrodes in X or Y direction it is possible to produce different skin treatment patterns by different carriers and by changing the size and distance between electrodes 604 and 608 it is possible to affect the depth of the treated skin layer. Electrodes 604 and 608 could be made of a rigid, semi-rigid or resilient electrically conducting material. Resilient electrodes conform to the skin relief more easily and enable better contact with the treated skin segment than the rigid electrodes enable. Resilient electrodes may be produced by coating the copper electrodes by a conductive and resilient coating such as for example a conductive silicone. The electrodes may be arranged in an array such as that shown in FIG. 7 where more than one electrode 704 share a common return electrode 728. Numeral 700 marks the substrate on which the electrodes are located. Other configurations adapted to treatment of different skin segments are possible.

The dimensions of the carriers illustrated above on which the arrays of corresponding voltage to skin applying elements or electrodes are mounted or assembled determines the size of the affected skin surface of the treated skin segment. Current sizes of carriers bearing the voltage applying elements range from 5 x 5 mm2 to 25 x 25 mm2 or 30X30 mm2. The affected skin surface is generally equal to the size of the carrier on which the electrodes are mounted. The arrays of electrodes may be located on the surface of the carriers, as an evenly or randomly spaced matrix of, for example but not limited to, 2X2 electrodes, 4X4 electrodes, 12 x 12 electrodes, 16 x 16 electrodes, 16 x 24 electrodes, or any other number and configuration of the electrodes. Concerning the various embodiments, the term randomly is intended to include true randomness, as well as pseudo randomness or even predictive sequencing of the operating electrodes with a variety of sequences.

Carrier design and size may be scaled-up or scaled down as desired or optimal for a particular treatment or size of area to be treated. For example, for a cosmetic treatment of different body segments the carrier may have a size of 60x60mm or more. Generally, the ability of supplying proper treatment power may be the size limiting factor. The diameter of the electrodes 208, 308 and 408 may be about 0.1 to 2 mm (about 100 to 2000 micron) and their size is usually selected such as to avoid formation of high density currents that may lead to skin ablation formation. In some embodiments, the electrodes may be configured in a pattern adapted to treat certain skin area having an irregular shape or surface.

RF voltage is proper coupled to skin when the contact between the skin and RF coupling electrode is such that most of the electrode surface is in contact with the skin. Electrodes being in partial contact with the skin may cause overheating of the treated skin segment; damage it and even initiate ablation. Apparatus 100 includes an electrode-to-skin contact detecting mechanism 136 (FIG. 1) enabling detection and operation only of electrodes that are in proper contact with the treated skin segment. The electrode to skin contact detection mechanism may be such as the one disclosed in Patent Cooperation Treaty Publication WO 2010/029536 to the same assignee and to the same inventors and in United States Patent No. 6,889,090 to the same assignee. The method disclosed in these applications assesses the quality of the contact between the electrodes and skin by monitoring skin impedance between the electrodes. The impedance measurement is an excellent indicator of the contact quality. Low impedance between the electrodes and the skin 212 (FIG. 2) means that firm contact between the electrode and the skin exists and accordingly the RF power is proper coupled to the skin. Continuous impedance monitoring provides continuous information input on the electrode-to-skin contact quality.

As described above, mechanism 136 (FIG. 1) is operative to detect and communicate to controller 112 only the electrodes that are in proper contact with the skin. Additionally, mechanism 136 may also serve, as will be explained below, to limit the number of active electrodes participating in the treatment. For example, a predetermined value of the impedance may be established as a part of treatment protocol. Controller 112 may not allow supply of RF voltage to electrodes with impedance values exceeding the predetermined value of the impedance.

Adapting the aesthetic treatment for personal use in the home environment requires automating the control of skin treatment parameters and introducing safety features to avoid over treatment of any particular segment of skin. Typically, the selected protocol and skin treatment parameters ensure safe skin treatment, which may be non-ablative skin treatment. Additionally, features such as, but not limited to, limiting the number of the voltage to skin application elements or electrodes activated at any one time, providing electrodes with relatively large contact surface, gradual application and/or activation of the power provided to skin application elements in a predetermined specific sequence may all contribute to the safety and comfort of the home aesthetic skin treatment, and ensure safe skin treatment.

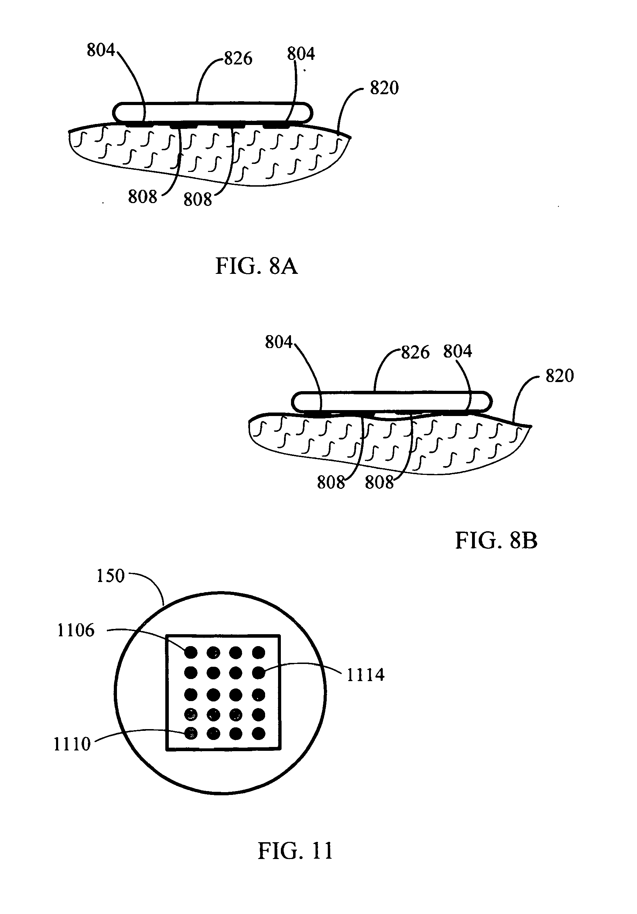

When one of the carriers described above is applied to a segment of skin 810 to be treated not all electrodes as shown in FIGS. 8A and 8B, which are schematic illustrations of full and insufficient contact of the electrode with a segment of skin, contact the skin simultaneously and with the same degree of contact (e.g., partial contact versus full contact). FIG. 8A illustrates a case where all electrodes 804 and 808 of tip 826 are in full contact with skin 820. FIG. 8B illustrates a case where electrodes 804 are in full contact with skin 820, one of the electrodes 808 is in partial contact with skin 820 and one of the electrodes 808 has no contact with skin 820. In order to limit the power coupled to the skin and avoid skin damage by improperly coupled electrodes, not all electrodes may be operative simultaneously. The skin-to-electrode contact detecting mechanism 136 may be used to determine the first four, five, ten or more electrodes being in proper (full) contact with the skin. The mechanism may communicate the electrode number or location in the matrix to controller 120 enabling operation of only the selected electrodes that are in proper contact with the treated skin segment and disabling operation of all other electrodes, preventing skin segment overheating and reducing possibility of undesired skin damage.

Skin conditions are unique and specific to each subject and different subjects have skins segments with different skin conditions. Even the skin of the same subject usually has segments with are different skin conditions. For example, some segments of the skin may be wet or dry, semi-wet skin and others. The skin conditions may affect the impedance of the treated skin segment. Skin conditions may be different even between the pairs of electrodes. Heating of wet skin employing RF energy reduces tissue impedance and may lead to skin temperature increase over a desired limit. Dry skin may require significant increase in the voltage to establish an RF induced current capable of heating the skin. US Provisional patent Application serial number 61/367,431 to the same Assignee teaches automatic adaptation of RF voltage or RF power applied to the skin.

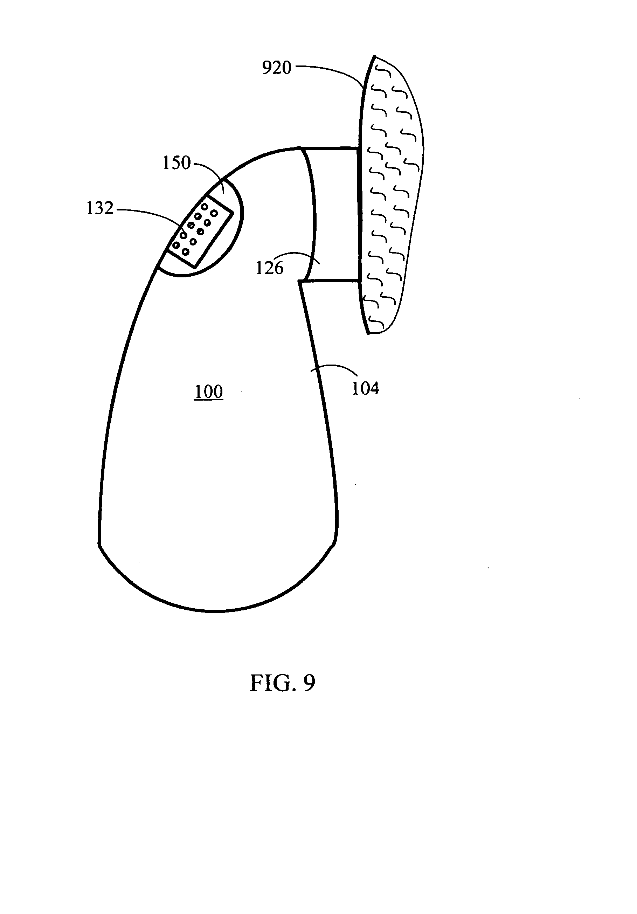

FIG 9 is a schematic illustration of an exemplary skin treatment process with the present apparatus. In order to treat his/her skin the subject couples/applies to a segment of skin to be treated any one of the carriers bearing a plurality of electrodes as described above against surface 920 of skin at a force level sufficient to ensure full (satisfactory) contact of one or more electrodes with surface 920 of the skin. A test RF voltage, which usually does not exceed 50 volt, is supplied to the electrodes. The electrode-to-skin contact status detection mechanism 136 detects the electrodes being in proper for treatment contact with the skin surface 920 and communicates the status of each of the electrodes to controller 112 (FIG. 1). Mechanism 156 becomes operative to retrieve from memory 160 appropriate skin treatment protocol and set the treatment parameters according to the retrieved from memory 160 skin treatment protocol.

The retrieved from memory 160 skin treatment protocol and treatment parameters may be applied to heat the treated skin segment by delivering to the selected electrodes a skin treatment RF voltage. There may be any number of selected electrodes for example one electrode, four electrodes, seven or more electrodes provided the number of the selected electrodes ensures safe treatment. Typically, the number of activated electrodes would be at least four or six or even more electrodes. If a carrier such as carrier 628 is applied to the skin, the RF induced current heats a skin layer equidistantly spaced from the skin surface or stratum corneum skin layer. The applied RF voltage would typically be between 50v to 400v. Typical RF frequency would be in the range of 350 KHz to 10MHz.

Upon completion of the treatment by one of the groups of electrodes controller 112 deactivates the just active group of electrodes and activates another group of electrodes without displacing apparatus 100 from the treated skin segment.

Different electrode selection criteria may be applied to the treatment based on a predetermined treatment protocol and input from the electrode-to-skin contact detection mechanism. The criteria may include the number of adjacent simultaneously active electrodes, randomly located electrodes and others. The controller may monitor the current flowing through the activated electrodes to ensure the activation of only the desired electrode at any one time, where the remaining electrodes are not operative at that time.

As mentioned above, the temperature of the different treated skin segments being in contact or located in the vicinity of the active electrodes may be monitored to avoid sudden temperature raise and deviation from the currently applied skin treatment protocol. Skin temperature increase over the temperature set by the treatment protocol may cause conversion of the skin heating process into a skin ablation process. In order to avoid this conversion a skin ablation detecting mechanism may monitor the skin treatment status and communicate the status reading or information to controller 112 (FIG. 1). Based on the skin treatment status provided by the skin temperature measurement mechanism or skin ablation detecting mechanism, controller 112 may change the skin treatment mode by changing the power of the applied RF radiation.

Treatment of skin by RF power may be further augmented by applying to skin light of suitable wavelength. The suitable wavelengths may be between 600nm to 1600nm.

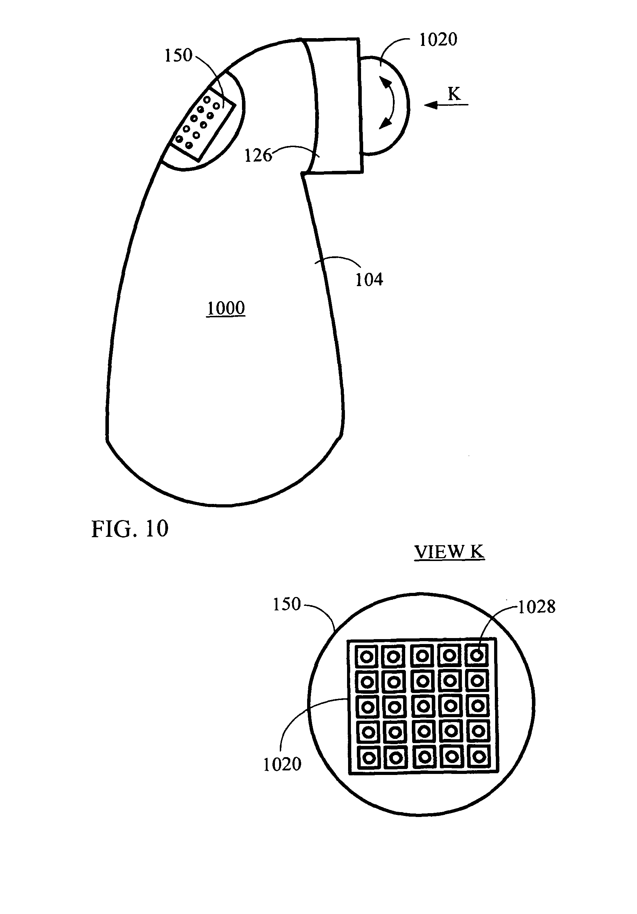

For treatment of the next target segment of skin, the apparatus may be moved (translated) over the skin in a patch-like step motion and be applied to the next target segment of skin to be treated. Alternatively, the apparatus could be moved in a sweep like movement over the treated skin segment. US Patent applications serial number 12/324,932 to the same assignee and to a common inventor discloses electrode carriers representing bodies with a rotational symmetry. In some embodiments of the present apparatus, as shown in FIG. 10, which is a simplified illustration of the present apparatus for personal aesthetic skin treatment with a tip implemented in the shape of a body with rotational symmetry, a similar carrier may be used with the present apparatus. Apparatus 1000 may be applied to the skin and be displaced on the skin in a continuous movement similar to paint brush movement or in a sweep like movement, for example, by rolling a roller 1020 bearing electrodes 1028 over a segment of skin to be treated. Apparatus 1000 could have an arrangement (not shown) or an encoder providing electric pulses related to the apparatus movement.

To achieve sufficient heat penetration into the dermis and satisfactory aesthetic treatment results, the electrodes are activated for a period of time in the range between 25msec and 10000msec. Other typical operating parameters of the apparatus may be: Voltage on high impedance load would be about 450Vpp causing a current of 50 400mA. The RF is usually supplied in pulse form with energy per pulse (Actual energy delivered to the skin) of 0.54J, more typical 12J. Generally, in home use devices employing low voltage settings there may be no need for cooling the electrodes or the treated skin segment and thus, almost all treatments may be performed with an apparatus that does not employ dedicated cooling means.

It should be understood that treatment applied with the above described apparatus is a noninvasive treatment. The contact elements or electrodes do not penetrate and do not introduce damage to the skin being treated. The RF voltage applied does not break through or damage the skin. Upon completion of the patient skin treatment, the carrier used to apply or distribute the voltage to the target segment of skin may be removed from the apparatus and be disposed of. It should also be noted that the exemplary carriers, although disposable upon completion of the treatment, may also be reused for a number of repeated treatments by the same subject.

Additionally and/or optionally as shown in FIG. 11, which is a simplified illustration of the display of the present apparatus indicating electrode status and in particular electrode-to-skin contact status in course of the aesthetic skin treatment process, the coupling of any one of the described above carriers against surface of skin at a force sufficient to ensure satisfactory contact of the electrodes with the surface of the skin may be monitored and displayed on display 150 (FIG. 1). Display 150 may include Organic Light Emitting Diodes (OLED), LCD or LED display each pixel of which dedicated to a specific electrode of the used carrier. Controller 112 (FIG. 1), which communicates with each one of the electrodes and display 150 corresponding OLEDs may display the contact status of each of the electrodes by illuminating the OLED dedicated to a specific electrode at a light color corresponding to the electrode contact and activity status. For example, one or more electrodes 1106 being in full contact with skin may be displayed in a green color, electrodes 1110 being in partial contact with the skin in orange and electrodes 1114 having no contact with the skin in red. Alternatively, an active electrode maybe displayed, for example, in white whereas a non-active electrode in blue.

The color OLED or other type of display may also provide other information such as display a map of the areas being treated at the time of the display as well as inoperable (defective) electrodes, such as electrodes receiving power but not generating heat in the skin volume below the electrode.

Full or proper contact between the electrodes and the skin may be improved and almost ensured by use of electrically conducting gel. Use of gel improves not only the contact of the electrodes with the skin, but also prevents skin ablation formation points.

For in home self-use apparatuses, the voltage applied to the treated segment of skin is sufficiently low to prevent ablation of the skin yet enabling sufficient generation of heat in the sub-epidermal layers to promote, for example, skin tightening and wrinkle reduction and other cosmetic treatments associated with collagen remodeling.

The present apparatus and method have been described using detailed descriptions of embodiments thereof that are provided by way of example and are not intended to limit the scope of the disclosure. The described embodiments comprise different features, not all of which are required in all embodiments of the apparatus. Some embodiments of the present apparatus and method utilize only some of the features or possible combinations of the features. Variations of embodiments of the present apparatus that are described and embodiments of the present method comprising different combinations of features noted in the described embodiments will occur to persons of the art.

## Claims

1. An apparatus for personal aesthetic skin treatment, said apparatus comprising:
a plurality of electrodes (132, 204, 208, 304, 404) operative to contact the skin and deliver to each contact RF voltage;
a mechanism (136) operative to detect quality of the contact between each of the electrodes and the skin;
a mechanism (158) operative to retrieve appropriate skin treatment protocol and set treatment parameters according to the retrieved skin treatment protocol;
said apparatus **characterized in that**
the mechanism (136) operative to detect the quality of the contact between each of the electrodes (132, 204, 208, 304, 404) and the skin also determines the number of electrodes (132, 204, 208, 304, 404) being in proper contact with the skin.

2. The apparatus according to claim 1, further comprising an RF generator (116) operative to supply the RF voltage independently and individually to each of the electrodes (132, 204, 208, 304, 404) according to the treatment protocol and wherein the RF voltage supplied is at least one of a group of voltages consisting of a RF test voltage and a skin treatment voltage.

3. The apparatus according to claim 2, wherein the RF test voltage is below 50 volt and wherein the skin treatment RF voltage is between 50V to 400V and wherein the test voltage does not exceed 50V.

4. The apparatus according to claim 1, further comprising a controller (112) including a mechanism (158) having a memory (160), the memory (160) stores and retrieves different skin treatment protocols and sets the skin treatment parameters according to the retrieved skin treatment protocol.

5. The apparatus according to claim 1, further comprising at least one electrode-to-skin quality of contact detecting mechanism (136) monitoring skin impedance between the electrodes (132, 204, 208, 304, 404) and at least one temperature sensor (240), said contact detecting mechanism (136) and said sensor (240) operative to provide to a controller (112) electrode-to-skin contact status and treated skin temperature.

6. The apparatus according to claim 1, wherein the apparatus also comprises a carrier including:
a substrate (200, 300, 400);
a return electrode (204, 308) surrounding said plurality of electrodes which are arranged as an array of electrodes (208, ,304, 404); and
a plurality of openings (412).

7. The apparatus according to claim 6, wherein said carrier is at least one of a group of carriers consisting of reusable (128, 328, 428) or disposable carriers and wherein the electrodes are at least one of a group consisting of resilient, rigid or semi-rigid electrodes.

8. The apparatus according to claim 6, wherein the openings (412) are configured and sized to accept LEDs (512) located on a tip of the apparatus and wherein the LEDs serve as registration pins for the disposable carrier.

9. The apparatus according to claim 1, wherein the electrodes (132, 208, 304, 404) have a diameter of about 100 µm to 2000µm.

10. The apparatus according to claim 1, wherein in course of skin treatment the plurality of electrodes (132, 208, 304, 404) are in contact with the skin at a plurality of skin locations.

11. The apparatus according to claim 1, further comprising a temperature sensor (240) operative to measure temperature of each of the electrodes, a group of the electrodes and the skin and communicate the temperature to a controller (112).

12. The apparatus according to claim 1, further comprising at least one light source with wavelength between 600nm to 1600nm operative to illuminate the treated skin segment.

13. The apparatus according to claim 1 further comprising a skin ablation detecting mechanism (144) operative to monitor the ongoing skin treatment status and detect situations in which ablation has been initiated.

14. The apparatus according to claim 1, further comprising a display (150) operative to provide information on the skin treatment process progress and a graphic map of the last treatment application.

15. The apparatus according to claim 14 wherein the display (150) is at least one of a group of displays consisting of Organic Light Emitting Diodes display, a Liquid Crystal Display and a matrix of Light Emitting Diodes and wherein the display displays for each electrode electrode-to-skin contact status.

## Patentansprüche

1. Vorrichtung zur persönlichen Hautschönheitsbehandlung, wobei die Vorrichtung Folgendes umfasst:
eine Mehrzahl von Elektroden (132, 204, 208, 304, 404), die betriebsfähig sind, um in Kontakt mit der Haut zu gelangen und jede Kontaktstelle mit RF-Spannung zu versorgen;
einen Mechanismus (136), der betriebsfähig ist, um die Qualität des Kontakts zwischen den einzelnen Elektroden und der Haut zu erkennen;
einen Mechanismus (158), der betriebsfähig ist, um das passende Hautbehandlungsprotokoll abzurufen und Behandlungsparameter entsprechend dem abgerufenen Hautbehandlungsprotokoll einzustellen;
wobei die Vorrichtung **dadurch gekennzeichnet ist, dass**
der Mechanismus (136), der betriebsfähig ist, um die Qualität des Kontakts zwischen den einzelnen Elektroden (132, 204, 208, 304, 404) und der Haut zu bestimmen, auch die Anzahl der Elektroden (132, 204, 208, 304, 404) bestimmt, die in ausreichendem Kontakt mit der Haut sind.

2. Vorrichtung nach Anspruch 1, ferner umfassend einen RF-Generator (116), der betriebsfähig ist, die einzelnen Elektroden (132, 204, 208, 304, 404) entsprechend dem Behandlungsprotokoll unabhängig und individuell mit RF-Spannung zu versorgen, und wobei die zugeführte RF-Spannung wenigstens eine von einer Gruppe von Spannungen ist, die aus einer RF-Prüfspannung und einer Hautbehandlungsspannung besteht.

3. Vorrichtung nach Anspruch 2, wobei die RF-Prüfspannung unter 50 Volt liegt und wobei die Hautbehandlungs-RF-Spannung zwischen 50 und 400 V liegt und die Prüfspannung 50 V nicht übersteigt.

4. Vorrichtung nach Anspruch 1, ferner umfassend eine Steuereinrichtung (112), die einen Mechanismus (158) mit einem Speicher (160) aufweist, wobei der Speicher (160) verschiedene Hautbehandlungsprotokolle speichert und abruft und die Hautbehandlungsparameter gemäß dem abgerufenen Hautbehandlungsprotokoll einstellt.

5. Vorrichtung nach Anspruch 1, ferner umfassend wenigstens einen Elektrode-Haut-Kontaktqualitätserkennungsmechanismus (136), der die Hautimpedanz zwischen den Elektroden (132, 204, 208, 304, 404) und wenigstens einem Temperatursensor (240) überwacht, wobei der Kontakterkennungsmechanismus (136) und der Sensor (240) betriebsfähig sind, Elektrode-Haut-Kontaktstatus und Temperatur der behandelten Haut an eine Steuereinrichtung (112) bereitzustellen.

6. Vorrichtung nach Anspruch 1, wobei die Vorrichtung außerdem einen Träger umfasst, aufweisend:
ein Substrat (200, 300, 400);
eine Rückelektrode (204, 308), die die Mehrzahl von Elektroden (208, 304, 404), die als ein Array angeordnet sind, umgibt; und
eine Mehrzahl von Öffnungen (412).

7. Vorrichtung nach Anspruch 6, wobei der Träger wenigstens einer von einer Gruppe von Trägern ist, die aus wiederverwendbaren (128, 328, 428) oder Einmalträgern besteht, und wobei die Elektroden wenigstens eins von einer Gruppe sind, die aus flexiblen, starren oder halbstarren Elektroden besteht.

8. Vorrichtung nach Anspruch 6, wobei die Öffnungen (412) dazu konfiguriert und ausgemessen sind, LEDs (512) aufzunehmen, die an einer Spitze der Vorrichtung angeordnet sind, und wobei die LEDs als Aufsetzstifte für den Einmalträger dienen.

9. Vorrichtung nach Anspruch 1, wobei die Elektroden (132, 208, 304, 404) einen Durchmesser von etwa 100 µm bis 2000 µm aufweisen.

10. Vorrichtung nach Anspruch 1, wobei im Zuge der Hautbehandlung die Mehrzahl von Elektroden (132, 208, 304, 404) an einer Mehrzahl von Hautstellen in Kontakt mit der Haut steht.

11. Vorrichtung nach Anspruch 1, ferner umfassend einen Temperatursensor (240), der betriebsfähig ist, um die Temperatur der einzelnen Elektroden, einer Gruppe der Elektroden und der Haut zu messen und die Temperatur an eine Steuereinrichtung (112) zu übermitteln.

12. Vorrichtung nach Anspruch 1, ferner umfassend wenigstens eine Lichtquelle mit Wellenlänge zwischen 600 nm und 1600 nm, die betriebsfähig ist, um das behandelte Hautsegment zu beleuchten.

13. Vorrichtung nach Anspruch 1, ferner umfassend einen Hautabtragungserkennungsmechanismus (144), der betriebsfähig ist, um den laufenden Hautbehandlungsstatus zu überwachen und Situationen zu erkennen, in der eine Abtragung eingeleitet wurde.

14. Vorrichtung nach Anspruch 1, ferner umfassend eine Anzeige (150), die betriebsfähig ist, um Informationen zum Fortschritt des Hautbehandlungsprozesses und eine Kartengrafik der letzten Behandlungsanwendung bereitzustellen.

15. Vorrichtung nach Anspruch 14, wobei die Anzeige (150) wenigstens eine von einer Gruppe von Anzeigen ist, bestehend aus einer organischen Leuchtdiodenanzeige, einer Flüssigkristallanzeige und einer Matrix aus Leuchtdioden, und wobei die Anzeige für jede Elektrode den Elektrode-Haut-Kontaktstatus anzeigt.

## Revendications

1. Appareil destiné au traitement personnel esthétique de la peau, ledit appareil comprenant :
une pluralité d'électrodes (132, 204, 208, 304, 404) servant à venir en contact avec la peau et à fournir à chaque contact une tension RF ;
un mécanisme (136) servant à détecter la qualité du contact entre chacune des électrodes et la peau ;
un mécanisme (158) servant à recueillir un protocole approprié de traitement de la peau et à établir des paramètres de traitement en fonction du protocole recueilli de traitement de la peau ;
ledit appareil **se caractérisant en ce que**
le mécanisme (136) servant à détecter la qualité du contact entre chacune des électrodes (132, 204, 208, 304, 404) et la peau détermine aussi le nombre d'électrodes (132, 204, 208, 304, 404) en contact approprié avec la peau.

2. Appareil selon la revendication 1, comprenant en outre un générateur de RF (116) servant à produire une tension RF indépendamment et individuellement aux bornes de chacune des électrodes (132, 204, 208, 304, 404) conformément au protocole de traitement et où la tension RF fournie est au moins l'une d'un groupe de tensions consistant en une tension d'essai de RF et une tension de traitement de la peau.

3. Appareil selon la revendication 2, dans lequel la tension d'essai RF est au-dessous de 50 V et où la tension RF de traitement de la peau est comprise entre 50 et 400 V et où la tension d'essai ne dépasse pas 50 V.

4. Appareil selon la revendication 1, comprenant en outre un contrôleur (112) comprenant un mécanisme (158) comportant une mémoire (160), la mémoire (160) stockant et récupérant différents protocoles de traitement de la peau et ajustant les paramètres de traitement de la peau selon le protocole recueilli de traitement de la peau.

5. Appareil selon la revendication 1, comprenant en outre au moins un mécanisme de détection de la qualité de contact électrode à peau (136) qui surveille l'impédance de la peau entre les électrodes (132, 204, 208, 304, 404) et au moins un capteur de température (240), ledit mécanisme de détection de contact (136) et ledit capteur (240) servant à procurer à un contrôleur (112) un statut du contact électrode à peau et une température de peau traitée.

6. Appareil selon la revendication 1, dans lequel l'appareil comprend aussi un support comprenant :
un substrat (200, 300, 400) ;
une électrode de retour (204, 308) entourant ladite pluralité d'électrodes qui sont disposées sous la forme d'un ensemble d'électrodes (208, 304, 404) ; et
un ensemble d'ouvertures (412).

7. Appareil selon la revendication 6, dans lequel ledit support est au moins un support d'un ensemble de supports constitué de supports réutilisables (128, 328, 428) ou jetables, et où les électrodes sont au moins une électrode d'un ensemble constitué d'électrodes résilientes, rigides ou semi-rigides.

8. Appareil selon la revendication 6, dans lequel les ouvertures (412) sont conçues et dimensionnées pour accepter des DEL (512) situées sur un embout de l'appareil et où les DEL servent de broches d'enregistrement pour le support jetable.

9. Appareil selon la revendication 1, dans lequel les électrodes (132, 208, 304, 404) ont un diamètre compris entre environ 100 et 2 000 µm.

10. Appareil selon la revendication 1, dans lequel en cours de traitement de la peau, la pluralité d'électrodes (132, 208, 304, 404) est en contact avec la peau au niveau d'une pluralité d'emplacements de la peau.

11. Appareil selon la revendication 1, comprenant en outre une sonde de température (240) servant à mesurer la température de chacune des électrodes, d'un groupe des électrodes et de la peau, et à communiquer la température à un contrôleur (112).

12. Appareil selon la revendication 1, comprenant en outre une source de lumière dont la longueur d'onde est comprise entre 600 et 1 600 nm servant à illuminer le segment de peau traité.

13. Appareil selon la revendication 1, comprenant en outre un mécanisme de détection d'ablation de la peau (144) servant à suivre le statut en cours du traitement de peau et à détecter des situations dans lesquelles l'ablation a été lancée.

14. Appareil selon la revendication 1, comprenant en outre un affichage (150) servant à donner des informations sur la progression du processus de traitement de la peau et une carte graphique de la dernière application du traitement.

15. Appareil selon la revendication 14, dans lequel l'affichage (150) est au moins un affichage d'un ensemble d'affichages constitué d'un affichage de diodes électroluminescentes organiques, d'un affichage à cristaux liquides et d'une matrice de diodes électroluminescentes, et où l'affichage affiche pour chaque électrode le statut du contact électrode à peau.
